# EUROPEAN PATENT SPECIFICATION

(11) **EP 3 024 475 B1**
(45) Date of publication and mention of the grant of the patent: **30.09.2020**
(21) Application number: 14721688.1
(22) Date of filing: 27.03.2014
(51) Int. Cl.: A61K 31/4709, A61K 31/496, A61K 31/573, A61K 31/58, A61K 45/06, A61K 9/00, A61K 38/14, A61P 27/02, A61P 31/04, A61P 31/00, A61P 29/00

(54) **PHARMACEUTICAL COMPOSITIONS FOR INTRAOCULAR ADMINISTRATION COMPRISING AN ANTIBACTERIAL AGENT AND AN ANTIINFLAMMATORY AGENT**
PHARMAZEUTISCHE ZUSAMMENSETZUNGEN ZUR INTRAOKULAREN VERABREICHUNG ENTHALTEND EINEN ANTIBAKTERIELLEN UND EINEN ENTZÜNDUNGSHEMMENDEN WIRKSTOFF
COMPOSITIONS POUR UNE ADMINISTRATION INTRAOCULAIRE COMPRENANT UN AGENT ANTIBACTÉRIEN ET UN AGENT ANTI-INFLAMMATOIRE

(30) Priority: 22.07.2013 US 201361958170 P
(43) Date of publication of application: 01.06.2016
(73) Proprietor: Harrow Health, Inc., San Diego, CA 92130 (US)
(72) Inventor: LIEGNER, Jeffery T., Newton, New Jersey 07860 (US); KAROLCHYK, John Scott, Lake Hopatcong, New Jersey 07849 (US); COVALESKY, Bernard, Randolph, New Jersey 07869 (US); DILZER, Richard, Long Valley, New Jersey 07853 (US); PETERS, Kallan, Flemington, New Jersey 08822 (US)
(74) Representative: Lee, Nicholas John
(86) International application number: PCT/US2014/032026
(87) International publication number: WO 2015/012899

(56) References cited:
- WO-A2-2011/049958
- Maxine Lipner: "Perioperative pharmacology. Bucking the drop trend", , May 2012 (2012-05), XP002727264, Retrieved from the Internet: URL:http://eyeworld.org/article-bucking-th e-drop-trend [retrieved on 2014-07-17]
- Liegner ET AL: "Transzonular Intravitreal Injection of Triamcinolone and Moxifloxicin After IOL Insertion in Lieu of Preop/Postop Eyedrops", , 24 April 2012 (2012-04-24), XP054975453, Retrieved from the Internet: URL:http://ascrs2012.conferencefilms.com/a tables.wcs?entryid=100047 [retrieved on 2014-07-16]
- MANGAN ET AL: "No endophthalmitis seen in case series of 'no-drop' cataract surgery", PRIMARY CARE OPTOMETRY NEWS,, vol. 18, no. 7, 1 July 2013 (2013-07-01), page 16, XP009179126,
- PAGANELLI FERNANDO ET AL: "A single intraoperative sub-tenon's capsule injection of triamcinolone and ciprofloxacin in a controlled-release system for cataract surgery.", INVESTIGATIVE OPHTHALMOLOGY & VISUAL SCIENCE JUL 2009, vol. 50, no. 7, July 2009 (2009-07), pages 3041-3047, XP002727265, ISSN: 1552-5783
- Diane Angelucci: "Intracameral injection studied to replace post-op eyedrops", , January 2006 (2006-01), XP002727322, Retrieved from the Internet: URL:http://www.eyeworld.org/article.php?si d=2918 [retrieved on 2014-07-16]
- ERMIS SITKI SAMET ET AL: "Treatment of Staphylococcus epidermidis endophthalmitis with intravitreal moxifloxacin in a rabbit model", TOHOKU JOURNAL OF EXPERIMENTAL MEDICINE, vol. 205, no. 3, March 2005 (2005-03), pages 223-229, XP002727266, ISSN: 0040-8727
- WISKUR BRANDT J ET AL: "Toward improving therapeutic regimens for Bacillus endophthalmitis.", INVESTIGATIVE OPHTHALMOLOGY & VISUAL SCIENCE APR 2008, vol. 49, no. 4, April 2008 (2008-04), pages 1480-1487, XP002727267, ISSN: 0146-0404
- SAKALAR YILDIRIM BAYEZIT ET AL: "Treatment of experimental Bacillus cereus endophthalmitis using intravitreal moxifloxacin with or without dexamethasone.", JOURNAL OF OCULAR PHARMACOLOGY AND THERAPEUTICS : THE OFFICIAL JOURNAL OF THE ASSOCIATION FOR OCULAR PHARMACOLOGY AND THERAPEUTICS DEC 2011, vol. 27, no. 6, December 2011 (2011-12), pages 593-598, XP002727268, ISSN: 1557-7732
- KUMAR BALAKRISHNA VINEETH ET AL: "Trans-zonular delivery of intravitreal triamcinolone acetonide in the management of pre-existing macular oedema during cataract surgery.", ACTA OPHTHALMOLOGICA SCANDINAVICA JUN 2006, vol. 84, no. 3, June 2006 (2006-06), pages 438-440, XP002727269, ISSN: 1395-3907
- RICHARD MANGAN: "Transzonular injection reduces need for post-operative eye drops", , 13 August 2013 (2013-08-13), XP002727270, Retrieved from the Internet: URL:http://nebraska.aoa.org/Documents/NE/F allConvention/OD/LenticularCOMNGTRIMOX2013 BW.pdf [retrieved on 2014-07-16]

## Description

### FIELD OF THE INVENTION

The present invention relates generally to the field of ophthalmology and more specifically to injectable ophthalmological compositions having anti-bacterial and anti-inflammatory properties, and to methods of preparing such compositions.

### BACKGROUND

In ophthalmological treatments and procedures, e.g., cataract surgery, pre- and post-operative eye drops are frequently used by the patients to eliminate or alleviate negative post-surgery complications such as infections, inflammation, and tissue edema. It has been reported that as many as 8% of all ocular surgery patients may suffer from infections, including the potentially catastrophic endophthalmitis, and various negative sight threatening side effects after surgery, such as inflammatory uveitis, corneal edema, and cystoid macular edema. Typically, the topical postoperative medications are prescribed for at-home use starting before and then after cataract surgery, and are typically self-administered, unless requiring a caregiver or family assistance.

These ophthalmic medication drops include anti-inflammatory and antibiotic agents and are highly effective, but require strict adherence to the treatment regimens, which is often difficult for many patients (with physical limitations or aversions to eyelid touching and manipulation) and is frequently expensive (well over $200 per procedure), causing patients' dissatisfaction. It is desirable to have an alternative procedure that would permit avoiding the necessity of the use of such post-surgery medications to save the associated post-operative trouble and expenses.

One such alternative procedure includes the intraoperative intravitreal injection by an atraumatic transzonular route that can achieve patient outcomes that are as good as, or better than, the current at-home eye drop regimen, removing the issues of compliance and medication administration accuracy. This patent specification discloses pharmaceutical compositions suitable for intraoperative ocular injections that can achieve such positive patient outcomes, and methods of fabricating and administering the same.

In the prior art, Maxine Lipner: "Perioperative pharmacology. Bucking the drop trend", May 2012 (2012-05), http://eyeworld.org/article-bucking-the-drop-trend discloses trans-zonular intravitreal injection of triamcinolone and moxifloxacin. In addition, Liegner et al, "Transzonular Intravitreal Injection of Triamcinolone and Moxifloxicin After IOL Insertion in Lieu of Preop/Postop Eyedrops", 24 April 2012, http://ascrs2012.conferencefilms.com/atables.wcs?entryid=100047 and Mangan et al, "No endophthalmitis seen in case series of 'nodrop' cataract surgery", Primary Care Optometry Care News, vol. 18, no. 7, 1 July 2013 page 16, disclose the trans-zonular injection of a mixture of triamcinolone acetonide and moxifloxacin.

### SUMMARY

A first aspect of the invention provides a pharmaceutical composition for intraocular injection, comprising (a) a therapeutic component consisting essentially of: (a1) a therapeutically effective quantity of an anti-bacterial agent, independently selected from the group consisting of a fluorinated quinolone and pharmaceutically acceptable salts, hydrates, solvates or N-oxides thereof; and (a2) a therapeutically effective quantity of an anti-inflammatory agent independently selected from the group consisting of corticosteroids and pharmaceutically acceptable salts, hydrates, solvates, ethers, esters, acetals and ketals thereof; (b) at least one pharmaceutically acceptable excipient suitable for intraocular injection; wherein at least one excipient is a solubilizing and suspending agent selected from the group consisting of non-ionic polyoxyethlene-polyoxypropylene block copolymers at a concentration of 0.01-1 mass %; and (c) optionally, a pharmaceutically acceptable carrier therefor.

In a second aspect, the present invention provides a method for preparing a pharmaceutical composition for intraocular injection comprising combining components (a) and (b) of the pharmaceutical composition of the first aspect to obtain the pharmaceutical composition thereby, optionally wherein the anti-bacterial agent, the anti-inflammatory agent, the excipient and the carrier are combined in a one-batch formulation method.

In a third aspect, the present invention provides the composition of the first aspect for use in a method for treating an ophthalmological disease, condition or pathology in a mammalian subject in need of such treatment comprising delivery to the subject the composition of the first aspect, wherein the method of delivery is selected from the group consisting of intravitreal injection, intraocular intracameral injection, intra-lesional injection, intra-articular injection, subconjunctival injection, sub-tenon injection, delivery via eye drops, delivery via spray and intra-canalicular delivery, to treat the ophthalmological disease, condition or pathology thereby.

In a fourth aspect, the present invention provides the composition of the first aspect for use in a method for treating an ophthalmological disease, condition or pathology in a mammalian subject in need of such treatment comprising intraocularly injecting the subject, wherein the composition comprises (a) a therapeutically effective quantity of moxifloxacin; (b) a therapeutically effective quantity of triamcinolone acetonide; and (c) a quantity of Poloxamer 407® to treat the ophthalmological disease, condition or pathology thereby.

In a fifth aspect, the present invention provide a pharmaceutical kit, comprising a sealed container containing the pharmaceutical composition of the first aspect and an instruction for the use of the composition enclosed with the container.

Preferred embodiments of each aspect of the invention are set out in the dependent claims herein.

The pharmaceutical compositions described herein may be intravitreally transzonularly injected into a mammalian subject as a part of the process of treatment of a variety of ophthalmological diseases, conditions or pathologies associated with intraocular surgery, such as cataracts, retinal and glaucoma disease.

### DETAILED DESCRIPTION

### A. Terms and Definitions

Unless specific definitions are provided, the nomenclatures utilized in connection with, and the laboratory procedures and techniques of analytical chemistry, synthetic organic and inorganic chemistry described herein, are those known in the art. Standard chemical symbols are used interchangeably with the full names represented by such symbols. Thus, for example, the terms "hydrogen" and "H" are understood to have identical meaning. Standard techniques may be used for chemical syntheses, chemical analyses, formulating compositions and testing them. The foregoing techniques and procedures can be generally performed according to conventional methods well known in the art.

It is to be understood that both the foregoing general description and the following detailed description are exemplary and explanatory only and are not restrictive of the invention claimed. As used herein, the use of the singular includes the plural unless specifically stated otherwise. The section headings used herein are for organizational purposes only and are not to be construed as limiting the subject matter described.

As used herein, "or" means "and/or" unless stated otherwise. Furthermore, use of the term "including" as well as other forms, such as "includes," and "included," is not limiting.

"About" as used herein means that a number referred to as "about" comprises the recited number plus or minus 1-10% of that recited number. For example, "about" 100 degrees can mean 95-105 degrees or as few as 99-101 degrees depending on the context. Whenever it appears herein, a numerical range such as "1 to 20" refers to each integer in the given range; i.e., meaning only 1, only 2, only 3, etc., up to and including only 20.

The term "pharmaceutical composition" is defined as a chemical or a biological compound or substance, or a mixture or combination of two or more such compounds or substances, intended for use in the medical diagnosis, cure, treatment,
or prevention of disease or pathology.

The term "intraocular injection" refers to an injection that is administered by entering the eyeball of the patient.

The term "transzonular" refers to an injection administered through the ciliary zonule which is a series of fibers connecting the ciliary body and lens of the eye.

The term "intravitreal" refers to an injection administered through an eye of the patient, directly into the inner cavity of the eye.

The term "intraoperative" is defined as an action occurring or carried during, or in the course of, surgery.

The terms "anti-bacterial" and "antibiotic" used herein interchangeably, refer to substances or compounds that destroy bacteria and/or inhibit the growth thereof via any mechanism or route.

The term "anti-inflammatory" refers to substances or compounds that counteract or suppress inflammation via any mechanism or route.

The term "quinolone" for the purposes of this application refers to a genus of anti-bacterial compounds that are derivatives of benzopyridine and in some embodiments include fluorine atom, such as in the following structure ("fluoroquinolone"):

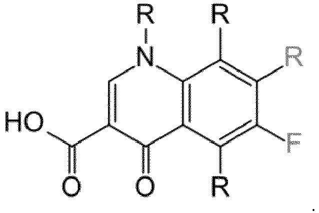

The term "corticosteroid" is defined as a compound belonging to a sub-genus of steroids that are derivatives of corticosterone, the latter having the chemical structure:

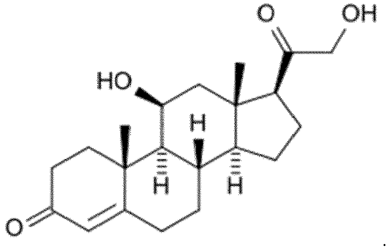

The term "salt" refers to an ionic compound which is a product of the neutralization reaction of an acid and a base.

The terms "solvate" and "hydrate" are used herein to indicate that a compound or a substance is physically or chemically associated with a solvent for "solvates" such as water (for "hydrates") .

The term "ether" refers to a chemical compound containing the structure R-O-Ri, where two organic fragments R and R₁ are connected via oxygen.

The term "ester" refers to a chemical compound containing the ester group R-O-C(O)-R₁, connecting two organic fragments R and R₁.

The terms "acetal" and "ketal" refer to a chemical compound containing the functional group R-C(R₁)(OR₂)₂, where R and R₂ are organic fragments and R₁ is hydrogen atom (for acetals), and is inclusive of "hemiacetals" where one R₂ (but not the other) is hydrogen atom; or where none of R, R₁ and R₂ is a hydrogen atom and each is an organic fragment (for ketals).

The term "carrier" refers to a substance that serves as a vehicle for improving the efficiency of delivery and the effectiveness of a pharmaceutical composition.

The term "excipient" refers to a pharmacologically inactive substance that is formulated in combination with the pharmacologically active ingredient of pharmaceutical composition and is inclusive of bulking agents, fillers, diluents and products used for facilitating drug absorption or solubility or for other pharmacokinetic considerations.

The term "therapeutically effective amount" is defined as the amount of the compound or pharmaceutical composition that will elicit the biological or medical response of a tissue, system, animal or human that is being sought by the researcher, medical doctor or other clinician.

The term "pharmaceutically acceptable" is defined as a carrier, whether diluent or excipient, that is compatible with the other ingredients of the formulation and not deleterious to the recipient thereof.

The terms "administration of a composition" or "administering a composition" is defined to include an act of providing a compound of the invention or pharmaceutical composition to the subject in need of treatment.

### B. Embodiments of the Invention

The concentration of the anti-bacterial agent in the pharmaceutical composition may be between about 0.01mg/mL and about 50.0 mg/mL, such as between about 0.5 mg/mL and about 10 mg/mL, for example, about 1.0 mg/mL. The concentration of the anti-inflammatory agent in the pharmaceutical composition may be between about 0.1mg/mL and about 100.0 mg/mL, such as between about 5.0 mg/mL and about 50.0 mg/mL, for example, about 15.0 mg/mL.

The anti-bacterial agent may be selected from the group of fluoroquinolones, and suitable derivatives of the same, such as pharmaceutically acceptable salts, hydrates, solvates, ethers, esters, acetals and ketals thereof. In one embodiment, the fluoroquinolone is moxifloxacin (chemically, 1-cyclopropyl-7-[(1S,6S)-2,8-diazabicyclo-[4.3.0]non-8-yl]-6-fluoro-8-methoxy-4-oxo-quinoline-3-carboxylic acid), which is available, e.g., under trade name Avelox® from Bayer Healthcare Corp. of Wayne, New Jersey, and under other trade names from other suppliers such as Alcon Corp. and Bristol-Myers Squibb Co. and has the following chemical structure:

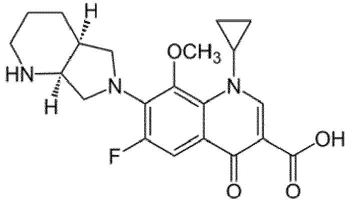

A non-limiting example of a possible alternative fluoroquinolone antibiotic that may be used instead of, or in combination with, moxifloxacin is gatifloxacin. One or several glycopeptide antibiotic(s), or a combination of some or all of them, may be optionally used as a part of the anti-bacterial agent, in combination with moxifloxacin. One example of such an acceptable additional glycopeptide antibiotic is vancomycin which can be introduced into the pharmaceutical composition at a concentration between about 1mg/mL and about 100.0 mg/mL, such as between about 5.0 mg/mL and about 50.0 mg/mL, for example, about 10.0 mg/mL. Vancomycin is available under the trade name Vancocin® from Eli Lilly & Co. of Indianapolis, Indiana. Other acceptable additional glycopeptide antibiotics that may be used include teicoplanin, telavancin, decaplanin, ramoplanin, gentamicin, tobramycin, amikacin, cefuroxime, polymyxin B sulfate, and trimethoprim.

The anti-inflammatory agent is selected from the group of corticosteroids, such as derivatives of corticosterone, and pharmaceutically acceptable salts, hydrates, solvates, ethers, esters, acetals and ketals thereof. For example, a product obtained as a result of a chemically reasonable substitution of any hydrogen and/or hydroxyl group in the molecule of corticosterone may be used. In one embodiment, a corticosteroid that can be so utilized is triamcinolone (chemically, (11β,16α)-9-fluoro-11,16,17,21-tetrahydroxypregna-1,4-diene-3,20-dione) having the following chemical formula:

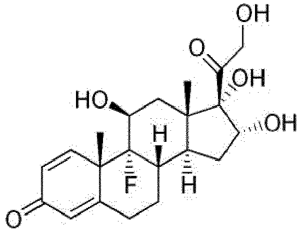

In another embodiment, a corticosteroid that can be so utilized is triamcinolone acetonide (chemically, (4aS,4bR,5S,6aS,6bS,9aR,10aS,10bS)-4b-fluoro-6b-glycoloyl-5-hydroxy-4a,6a,8,8-tetramethyl-4a,4b,5,6,6a,6b,9a,10,10a,10b,11,12-dodecahydro-2H-naphtho[2',1':4,5]indeno[1,2-d][1,3]dioxol-2-one) which is a ketal derivative of triamcinolone available, e.g., under the trade name Kenalog® from Bristol-Myers Squibb Co. of Princeton, New Jersey, and under other trade names from other suppliers, and having the following chemical formula:

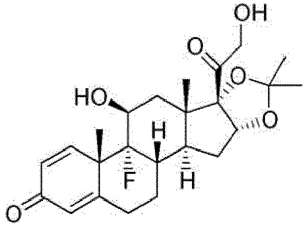

Other corticosteroids, or a combination of some or all of them, may be used instead of all or a portion of triamcinolone and/or of all or a portion of triamcinolone acetonide. Some non-limiting examples of such acceptable other corticosteroids include triamcinolone diacetate, triamcinolone benetonide, triamcinolone furetonide, triamcinolone hexacetonide, betamethasone acetate, dexamethasone, fluorometholone and fluocinolone acetonide.

As mentioned above, the pharmaceutical composition of the invention further includes one or several pharmaceutically acceptable excipient(s) which is a solubilizing and suspending agent selected from the group consisting of non-ionic polyoxyethlene-polyoxypropylene block copolymers at a concentration of 0.01-1 mass %. Those having ordinary skill in the art will be able to select the suitable excipient(s). It is worth mentioning that when moxifloxacin is used in pharmaceutical formulations, it is often difficult to obtain a stable suspension of another product (e.g., a corticosteroid such as triamcinolone acetonide) that is present in the same formulation and that needs to be in a form of a stable suspension. Without being bound by any particular scientific theory, such difficulties in obtaining the stable suspension are believed to be caused by moxifloxacin's tendency to deactivate many suspending agents resulting in unacceptable coagulation, clumping and flocculation. As a result, normal delivery through a typical 27-29 gage cannula is often difficult or even impossible.

Therefore, it is desirable to select an excipient that is stable in the presence of moxifloxacin and can, therefore, be used as a solubilizing and suspending agent to ensure that the corticosteroid such as triamcinolone acetonide safely forms a stable suspension even when moxifloxacin is also present in the same formulation. Numerous attempts by others to produce a stable moxifloxacin/triamcinolone acetonide pharmaceutical composition suitable for intraocular injection have not been successful.

An excipient that can be used as a solubilizing and stabilizing agent to overcome the above-described difficulties and thus to obtain a stable suspension of the corticosteroid such as triamcinolone acetonide may be a non-ionic polyoxyethlene-polyoxypropylene block copolymer having the following general structure:

HO-(CH₂-CH₂-O)ₓ-(C₃H₆-O)_{y}-(CH₂-CH₂-O)ₓ-H,

wherein x is an integer having the value of at least 8 and x is an integer having the value of at least 38.

The content of the non-ionic polyoxyethlene-polyoxypropylene block copolymer used as a solubilizing and stabilizing agent in the pharmaceutical compositions of the present invention is between 0.01 mass % and 1.0 mass %.

One non-limiting example of a specific non-ionic polyoxyethlene-polyoxypropylene block copolymer that can be used as a solubilizing and stabilizing agent in the pharmaceutical compositions of the presentinvention is the product known under the trade name Poloxamer 407® (poly(ethylene glycol)-block-poly(propylene glycol)-block-poly(ethylene glycol)) available from Sigma-Aldrich Corp. of St. Louis, Missouri, with the molecular weight of the polyoxypropylene portion of about 4,000 Daltons, about a 70% polyoxyethylene content, the overall molecular weight of between about 9,840 Daltons and about 14,600 Daltons and having the following chemical structure

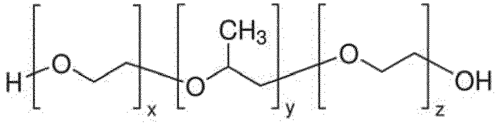

Non-limiting examples of some other excipients and carriers that may be used in preparing in the pharmaceutical compositions of the present invention include polysorbate (an emulsifier), edetate calcium disodium (EDTA, a chelating agent), hydrochloric acid (the pH adjuster) and sterile water.

According to a further aspect, methods for fabricating the above-described pharmaceutical compositions are provided. A one-batch formulation method may be used, where the components of the pharmaceutical formulation can be combined in single container; the components may be added to the container simultaneously or consecutively.

In one exemplary, non-limiting procedure, a quantity of an anti-bacterial agent such as moxifloxacin may be placed into a mixing container followed by adding a quantity of sterile water and hydrochloric acid to obtain a slightly acidic mixture (e.g., having pH of about 6.5) which is stirred until a clear solution is obtained. In case of moxifloxacin/HCl system, the solution is stable, allowing the formulation to remain closed system thus preventing contamination and the loss of sterility.

Next, a quantity of corticosteroid such as micronized triamcinolone acetonide, a quantity of Poloxamer 407®, a quantity of edetate calcium disodium and a quantity of polysorbate 80 may be all added to be combined in the same container with the already prepared moxifloxacin/HCl solution and stirred together (e.g., by spinning) for a period of time, e.g., about 6 hours, until a homogenous suspension has been obtained. The resulting suspension may then be transferred into single dose vials, capped, sealed, autoclaved and shaken until cool. Finally, a complete testing for sterility and the presence of endotoxin may be performed on the product according to commonly used methods known to those having ordinary skill in the art.

Pharmaceutical compositions prepared as described above can be used to prevent complications that may arise after ophthalmic surgical operations and procedure. For example, the formulations can be used during any intraocular surgery, such as cataract surgery, planned vitrectomy or glaucoma procedures, to prevent or at least substantially reduce the risk of post-surgery complications, such as the development of endophthalmitis or cystoid macular edema (CME), without having the patient use pre- or post-operative topical ophthalmic drops. Individuals with evidence of endophthalmitis from prior surgical procedures or traumatic ocular penetration will benefit from concurrent injection of these formulations to sterilize infection and reduce damaging inflammation.

Pharmaceutical formulations described herein can be delivered via intraocular intravitreal injection which can be transzonular, or, if desired not transzonular. Intraocular intravitreal injection of this formulation, whether done via transzonular or via direct pars plana (trans-scleral) injection, delivers potent broad spectrum antibiotics directly into the suppurative tissue without requiring the urgent compounding of multiple individual medications or multiple individual injections into the eye.

Typically, a pharmaceutical composition described above will be intraocularly administered to a mammalian subject (e.g., humans, cats, dogs, other pets, domestic, wild or farm animals) in need of emergent, urgent or planned ophthalmic surgery treatment. The effect achieved by such use of pharmaceutical composition described above may last up to four weeks. The composition is to be injected intravitreally and trans-zonularly using methods and techniques known to those having ordinary skilled in the art of ophthalmology. In some embodiments, the injection can be intraoperative.

Typically, the delivery through a typical 27 gauge cannula can be employed utilizing a 1 mL TB syringe, with attention to re-suspending the formulation using momentary flicks and shake just prior to injection. The medicinal volume (i.e., dosage) required of this formulation varies based on the type of intraocular procedure, the degree of postoperative inflammation induced or anticipated, the risk assessment for postoperative infection, and anatomic considerations regarding the available volume for the injection being added to a closed intraocular space.

It is worth mentioning that while intracameral (that is, anterior chamber) injections are within the scope of the present invention such injections instead of posterior chamber (intravitreal) injection may not be satisfactory in some cases, as the suspension clogs the trabecular meshwork and aggravates intraocular drainage, resulting in an intraocular pressure rise postoperative. This is avoided with intravitreal injection, in addition to retaining the formulation components into the protein matrix of the vitreous of a greater duration. Anterior chamber wash out occurs over hours (antibiotic in solution) and days (steroid in suspension), while intravitreal injection is retained for weeks.

In alternative embodiments, if desired or necessary the formulations may also be delivered in the form of eye drops or eye sprays, as well as via subconjunctival injection, intraocular intracameral injection, sub-tenon injection, intra-articular injection or intra-lesional injection, particularly, in, but not limited to, some cases when necessary to deliver additional medication when local ocular inflammation and extra-ocular infection need suppression. Intravitreal delivery of steroid has historically been used to treat clinically significant cystoid macular edema (CME); the application of this formulation into the vitreous during routine intraocular procedures brings more aggressive prophylaxis against CME occurrence. Additionally, the suspension of this formulation is useful for staining vitreous during planned and unplanned vitrectomies, improving visualization of this otherwise transparent intraocular tissue, improving vitrectomy outcomes and reducing complications resulting from inadequate or tractional vitreous removal. In still further embodiments, there is also envisioned intra-canalicular delivery, i.e., delivery via a lacrimal canaliculus implant.

Instead of delivering the above-described compositions comprising both anti-bacterial and anti-inflammatory agents, consecutive injections may be used instead, if desired. For example, triamcinolone may be injected first, immediately followed by the injection of moxifloxacin or vice versa.

It will be understood by those having ordinary skill in the art that the specific dose level and frequency of dosage for any particular patient may be varied and will depend upon a variety of factors including the activity of the specific compound employed, the metabolic stability and length of action of that compound, the age, body weight, general health, gender, diet, and the severity of the particular ophthalmological condition being treated.

Pharmaceutical kits are provided. The kit includes a sealed container approved for the storage of pharmaceutical compositions, the container containing one of the above-described pharmaceutical compositions. An instruction for the use of the composition and the information about the composition are to be included in the kit.

The following examples are provided to further elucidate the advantages and features of the present invention, but are not intended to limit the scope of the invention. The examples are for the illustrative purposes only. USP pharmaceutical grade products were used in preparing the formulations described below.

### C. Examples

### Example 1. Preparing a Pharmaceutical Composition

A pharmaceutical composition was prepared as described below. The following products were used in the amounts and concentrations specified:
(a) about 1.5 g of triamcinolone acetonide, at a concentration of about 15.0 mg/mL;
(b) about 0.1 g of moxifloxacin hydrochloride, at a concentration of about 1.0 mg/mL;
(c) about 1 mL of polysorbate 80, at a concentration of about 1.0 mass %;
(d) about 0.2 g of edetate calcium disodium, at a concentration of about 0.2 mass %;
(e) about 1 g of Poloxamer 407®, at a concentration of about 1.0 mass %;
(f) hydrochloric acid, to adjust pH to about 6.5; and
(g) about 100.0 mL of sterile water for injection.

Moxifloxacin hydrochloride was placed into a de-pyrogenated beaker with a spin bar. Sterile water for injection was added to about 1/3 of the volume of the beaker. While spinning, moxifloxacin was dissolved by adding hydrochloric acid until a clear solution having the final pH of about 6.5 was obtained.

The solution was combined with micronized triamcinolone acetonide, Poloxamer 407®, edetate calcium disoudium and polysorbate 80 and allowed to spin for about 6 hours until a hydrated and homogenous suspension was obtained.

The suspension was transferred into de-pyrogenated, single dose vials (2mL size), capped and sealed, followed by autoclaving and shaking the vials until cool. Complete sterility and endotoxin testing was performed by an outside laboratory to ensure safety.

The formulation prepared as described above was tested for stability after 6 months of storage. After this period of storage no loss of potency was observed (as measured by HPLC); the formulation was visually stable at room temperature and readily re-suspended with gentle shaking with no increase of particle size or flocculation.

### Example 2. Preparing a Pharmaceutical Composition Containing Vancomycin

A pharmaceutical composition was prepared as described in Example 1, *supra.* The composition was autoclaved and sonicated for about 60 minutes and about 96 mL of the composition were combined with about 4 mL of vancomycin at a concentration of about 250 mg/mL. The pH of the mixture was adjusted to about 6.0-6.5 using hydrochloric acid. The product was then transferred into vials (at about 1 mL plus 5 drops per vial) and frozen. The product has kept its stability and potency for at least six months.

### Example 3. Using a Pharmaceutical Composition

A pharmaceutical composition fabricated as described in Example 1, *supra,* was administered to about 1,600 patients. To each, it was introduced using intravitreal transzonular injection. The injection was intraoperative. Only a very few patients, at the rate of about only 1 in 4,000, have developed any infection or suffered from other side effects that required further treatment, which is a substantial improvement over a typical rate of about 8% for the patients that did not receive the injection.

Although the invention has been described with reference to the above examples, it will be understood that modifications and variations are encompassed within the scope of the invention. Accordingly, the invention is limited only by the following claims.

## Claims

1. A pharmaceutical composition for intraocular injection, comprising
(a) a therapeutic component consisting essentially of:
(a1) a therapeutically effective quantity of an anti-bacterial agent, independently selected from the group consisting of a fluorinated quinolone and pharmaceutically acceptable salts, hydrates, solvates or N-oxides thereof; and
(a2) a therapeutically effective quantity of an anti-inflammatory agent independently selected from the group consisting of corticosteroids and pharmaceutically acceptable salts, hydrates, solvates, ethers, esters, acetals and ketals thereof;
(b) at least one pharmaceutically acceptable excipient suitable for intraocular injection; wherein at least one excipient is a solubilizing and suspending agent selected from the group consisting of non-ionic polyoxyethlene-polyoxypropylene block copolymers at a concentration of 0.01 - 1 mass%; and
(c) optionally, a pharmaceutically acceptable carrier therefor.

2. The pharmaceutical composition of claim 1, wherein the composition is for delivery in the form of eye drops or eye sprays.

3. The pharmaceutical composition of claim 1 or 2, wherein the fluorinated quinolone is selected from the group consisting of moxifloxacin and gatifloxacin.

4. The pharmaceutical composition of any preceding claim, wherein the corticosteroid is selected from the group consisting of triamcinolone, triamcinolone acetonide, triamcinolone diacetate, triamcinolone benetonide, triamcinolone furetonide, triamcinolone hexacetonide, betamethasone acetate, dexamethasone, fluorometholone, fluocinolone acetonide and a combination thereof.

5. The pharmaceutical composition of any preceding claim, wherein:
(a) the anti-bacterial agent is moxifloxacin; and/or
(b) the corticosteroid is triamcinolone or a pharmaceutically acceptable salt, hydrate, solvate, ether, ester, acetal or ketal thereof.

6. The pharmaceutical composition of any preceding claim, wherein the non-ionic polyoxyethlene-polyoxypropylene block copolymer is Poloxamer 407®.

7. The pharmaceutical composition of any preceding claim, comprising:
(a) moxifloxacin at a concentration of about 1.0 mg/mL;
(b) triamcinolone acetonide at a concentration of about 15.0 mg/mL; and
(c) Poloxamer 407® at a concentration of about 1.0 mass %.

8. The pharmaceutical composition of any preceding claim, further comprising a therapeutically effective quantity of an antibiotic selected from the group consisting of vancomycin, teicoplanin, telavancin, decaplanin, ramoplanin, gentamicin, tobramycin, amikacin, cefuroxime, polymyxin B sulfate, trimethoprim, and a combination thereof.

9. A method for preparing a pharmaceutical composition for intraocular injection comprising combining components (a) and (b) of any preceding claim to obtain the pharmaceutical composition thereby, optionally wherein the anti-bacterial agent, the anti-inflammatory agent, the excipient and the carrier are combined in a one-batch formulation method.

10. The composition of any one of claims 1 to 7 for use in a method for treating an ophthalmological disease, condition or pathology in a mammalian subject in need of such treatment comprising delivery to the subject the composition of claim 1, wherein the method of delivery is selected from the group consisting of intravitreal injection, intraocular intracameral injection, intra-lesional injection, intra-articular injection, subconjunctival injection, sub-tenon injection, delivery via eye drops, delivery via spray and intra-canalicular delivery, to treat the ophthalmological disease, condition or pathology thereby.

11. The composition for use of claim 10, wherein the intravitreal injection is selected from the group consisting of a transzonular injection and a non-transzonular injection.

12. The composition for use of claim 10, wherein the composition is injected intravitreally transzonularly.

13. The composition for use of claim 12, wherein (i) the anti-bacterial agent is moxifloxacin; and/or the corticosteroid is triamcinolone or a pharmaceutically acceptable salt, hydrate, solvate, ether, ester, acetal or ketal thereof or (ii) moxifloxacin at a concentration of about 1.0 mg/mL; triamcinolone acetonide at a concentration of about 15.0 mg/mL; and Poloxamer 407® at a concentration of about 1.0 mass %.

14. The composition of claim 2 for use in a method for treating an ophthalmological disease, condition or pathology in a mammalian subject in need of such treatment comprising intraocularly injecting the subject, wherein the composition comprises
(a) a therapeutically effective quantity of moxifloxacin;
(b) a therapeutically effective quantity of triamcinolone acetonide; and
(c) a quantity of Poloxamer 407®
to treat the ophthalmological disease, condition or pathology thereby.

15. The composition for use of claim 14, wherein the injecting is intravitreal and transzonular, or the injecting is intraoperative.

16. A pharmaceutical kit, comprising a sealed container containing the pharmaceutical composition of any one of claims 1 to 7 and an instruction for the use of the composition enclosed with the container.

## Patentansprüche

1. Pharmazeutische Zusammensetzung für die intraokulare Injektion, umfassend
(a) eine therapeutische Komponente, bestehend im Wesentlichen aus:
(a1) einer therapeutisch wirksamen Menge eines antibakteriellen Mittels, unabhängig ausgewählt aus der Gruppe bestehend aus einem fluorierten Chinolon und pharmazeutisch unbedenklichen Salzen, Hydraten, Solvaten oder N-Oxiden davon, und
(a2) einer therapeutisch wirksamen Menge eines entzündungshemmenden Mittels, unabhängig ausgewählt aus der Gruppe bestehend aus Corticosteroiden und pharmazeutisch unbedenklichen Salzen, Hydraten, Solvaten, Ethern, Estern, Acetalen und Ketalen davon,
(b) mindestens einen für die intraokulare Injektion geeigneten pharmazeutisch unbedenklichen Exzipienten, wobei es sich bei mindestens einem Exzipienten um ein Solubilisierungs- und Suspensionsmittel ausgewählt aus der Gruppe bestehend aus nichtionischen Polyoxyethylenpolyoxypropylen-Blockcopolymeren in einer Konzentration von 0,01-1 Massen-% handelt, und
(c) gegebenenfalls einen pharmazeutisch unbedenklichen Träger dafür.

2. Pharmazeutische Zusammensetzung nach Anspruch 1, wobei die Zusammensetzung für die Verabreichung in Form von Augentropfen oder Augensprays bestimmt ist.

3. Pharmazeutische Zusammensetzung nach Anspruch 1 oder 2, wobei das fluorierte Chinolon aus der aus Moxifloxacin und Gatifloxacin bestehenden Gruppe ausgewählt ist.

4. Pharmazeutische Zusammensetzung nach einem der vorhergehenden Ansprüche, wobei das Corticosteroid aus der aus Triamcinolon, Triamcinolonacetonid, Triamcinolondiacetat, Triamcinolonbenetonid, Triamcinolonfuretonid, Triamcinolonhexacetonid, Betamethasonacetat, Dexamethason, Fluorometholon, Fluocinolonacetonid und einer Kombination davon bestehenden Gruppe ausgewählt ist.

5. Pharmazeutische Zusammensetzung nach einem der vorhergehenden Ansprüche, wobei:
(a) es sich bei dem antibakteriellen Mittel um Moxifloxacin handelt und/oder
(b) es sich bei dem Corticosteroid um Triamcinolon oder ein pharmazeutisch unbedenkliches Salz, ein pharmazeutisch unbedenkliches Hydrat, ein pharmazeutisch unbedenkliches Solvat, einen pharmazeutisch unbedenklichen Ether, einen pharmazeutisch unbedenklichen Ester, ein pharmazeutisch unbedenkliches Acetal oder ein pharmazeutisch unbedenkliches Ketal davon handelt.

6. Pharmazeutische Zusammensetzung nach einem der vorhergehenden Ansprüche, wobei es sich bei dem nichtionischen Polyoxyethylen-polyoxypropylen-Blockcopolymer um Poloxamer 407® handelt.

7. Pharmazeutische Zusammensetzung nach einem der vorhergehenden Ansprüche, umfassend:
(a) Moxifloxacin in einer Konzentration von etwa 1,0 mg/ml,
(b) Triamcinolonacetonid in einer Konzentration von etwa 15,0 mg/ml und
(c) Poloxamer 407® in einer Konzentration von etwa 1,0 Massen-%.

8. Pharmazeutische Zusammensetzung nach einem der vorhergehenden Ansprüche, weiterhin umfassend eine therapeutisch wirksame Menge eines Antibiotikums ausgewählt aus der Gruppe bestehend aus Vancomycin, Teicoplanin, Telavancin, Decaplanin, Ramoplanin, Gentamicin, Tobramycin, Amikacin, Cefuroxim, Polymyxin-B-sulfat, Trimethoprim und einer Kombination davon.

9. Verfahren zur Herstellung einer pharmazeutischen Zusammensetzung für die intraokulare Injektion, bei dem man die Komponenten (a) und (b) nach einem der vorhergehenden Ansprüche unter Erhalt der pharmazeutischen Zusammensetzung zusammengibt, wobei das antibakterielle Mittel, das entzündungshemmende Mittel, der Exzipient und der Träger gegebenenfalls in einem Einschargenformulierungsverfahren zusammengegeben werden.

10. Zusammensetzung nach einem der Ansprüche 1 bis 7 zur Verwendung in einem Verfahren zur Behandlung einer ophthalmologischen Krankheit, eines ophthalmologischen Leidens oder einer ophthalmologischen Pathologie in einem einer solchen Behandlung bedürftigen Säugetiersubjekt, umfassend die Verabreichung der Zusammensetzung nach Anspruch 1 an das Subjekt, wobei die Verabreichungsmethode ausgewählt ist aus der Gruppe bestehend aus intravitrealer Injektion, intraokularer intrakameraler Injektion, intraläsionaler Injektion, intraartikulärer Injektion, subkonjunktivaler Injektion, Subtenoninjektion, Verabreichung mittels Augentropfen, Verabreichung mittels Spray und intrakanalikulärer Verabreichung, zur Behandlung der ophthalmologischen Krankheit, des ophthalmologischen Leidens bzw. der ophthalmologischen Pathologie dadurch.

11. Zusammensetzung zur Verwendung nach Anspruch 10, wobei die intravitreale Injektion aus der aus einer transzonulären Injektion und einer nichttranszonulären Injektion bestehenden Gruppe ausgewählt ist.

12. Zusammensetzung zur Verwendung nach Anspruch 10, wobei die Zusammensetzung intravitreal transzonulär injiziert wird.

13. Zusammensetzung zur Verwendung nach Anspruch 12, wobei (i) es sich bei dem antibakteriellen Mittel um Moxifloxacin handelt und/oder es sich bei dem Corticosteroid um Triamcinolon oder ein pharmazeutisch unbedenkliches Salz, ein pharmazeutisch unbedenkliches Hydrat, ein pharmazeutisch unbedenkliches Solvat, einen pharmazeutisch unbedenklichen Ether, einen pharmazeutisch unbedenklichen Ester, ein pharmazeutisch unbedenkliches Acetal oder ein pharmazeutisch unbedenkliches Ketal davon handelt oder (ii) Moxifloxacin in einer Konzentration von etwa 1,0 mg/ml, Triamcinolonacetonid in einer Konzentration von etwa 15,0 mg/ml und Poloxamer 407® in einer Konzentration von etwa 1,0 Massen-%.

14. Zusammensetzung nach Anspruch 2 zur Verwendung in einem Verfahren zur Behandlung einer ophthalmologischen Krankheit, eines ophthalmologischen Leidens oder einer ophthalmologischen Pathologie in einem einer solchen Behandlung bedürftigen Säugetiersubjekt, bei dem man das Subjekt intraokular injiziert, wobei die Zusammensetzung
(a) eine therapeutisch wirksame Menge an Moxifloxacin,
(b) eine therapeutisch wirksame Menge an Triamcinolonacetonid und
(c) eine Menge an Poloxamer 407®
umfasst, zur Behandlung der ophthalmologischen Krankheit, des ophthalmologischen Leidens bzw. der ophthalmologischen Pathologie.

15. Zusammensetzung zur Verwendung nach Anspruch 14, wobei die Injektion intravitreal und transzonulär erfolgt oder die Injektion intraoperativ erfolgt.

16. Pharmazeutisches Kit, umfassend einen die pharmazeutische Zusammensetzung nach einem der Ansprüche 1 bis 7 enthaltenden versiegelten Behälter und Anweisungen zum Gebrauch der in dem Behälter eingeschlossenen Zusammensetzung.

## Revendications

1. Composition pharmaceutique pour une injection intraoculaire, comprenant
(a) un composant thérapeutique essentiellement constitué de :
(a1) une quantité thérapeutiquement efficace d'un agent antibactérien, indépendamment choisi dans le groupe constitué par une quinolone fluorée et des sels, des hydrates, des solvates ou des N-oxydes pharmaceutiquement acceptables correspondants ; et
(a2) une quantité thérapeutiquement efficace d'un agent anti-inflammatoire indépendamment choisi dans le groupe constitué par des corticostéroïdes et des sels, des hydrates, des solvates, des éthers, des esters, des acétals et des cétals pharmaceutiquement acceptables correspondant ;
(b) au moins un excipient pharmaceutiquement acceptable approprié pour une injection intraoculaire ; au moins un excipient étant un agent solubilisant et de suspension choisi dans le groupe constitué par des copolymères à blocs polyoxyéthylène-polyoxypropylène non ioniques à raison d'une concentration de 0,01 à 1 % en masse ; et
(c) éventuellement, un support pharmaceutiquement acceptable pour celle-ci.

2. Composition pharmaceutique selon la revendication 1, la composition étant destinée à une administration sous forme de gouttes oculaires ou de sprays oculaires.

3. Composition pharmaceutique selon la revendication 1 ou 2, la quinolone fluorée étant choisie dans le groupe constitué par la moxifloxacine et la gatifloxacine.

4. Composition pharmaceutique selon une quelconque revendication précédente, le corticostéroïde étant choisi dans le groupe constitué par la triamcinolone, l'acétonide de triamcinolone, le diacétate de triamcinolone, le bénétonide de triamcinolone, le furétonide de triamcinolone, l'hexacétonide de triamcinolone, l'acétate de bêtaméthasone, la dexaméthasone, la fluorométholone, l'acétonide de fluocinolone et une combinaison correspondante.

5. Composition pharmaceutique selon une quelconque revendication précédente,
(a) l'agent antibactérien étant la moxifloxacine ; et/ou
(b) le corticostéroïde étant la triamcinolone ou un sel, un hydrate, un solvate, un éther, un ester, un acétal ou un cétal pharmaceutiquement acceptable correspondant.

6. Composition pharmaceutique selon une quelconque revendication précédente, le copolymère à blocs polyoxyéthylène-polyoxypropylène non ionique étant le Poloxamer 407®.

7. Composition pharmaceutique selon une quelconque revendication précédente, comprenant :
(a) de la moxifloxacine à une concentration d'environ 1,0 mg/mL ;
(b) de l'acétonide de triamcinolone à une concentration d'environ 15,0 mg/mL ; et
(c) du Poloxamer 407® à une concentration d'environ 1,0 % en masse.

8. Composition pharmaceutique selon une quelconque revendication précédente, comprenant en outre une quantité thérapeutiquement efficace d'un antibiotique choisi dans le groupe constitué par la vancomycine, la téicoplanine, la télavancine, la décaplanine, la ramoplanine, la gentamicine, la tobramycine, l'amikacine, le céfuroxime, le sulfate de polymyxine B, le triméthoprim, et une combinaison correspondante.

9. Procédé pour la préparation d'une composition pharmaceutique pour une injection intraoculaire comprenant la combinaison des composants (a) et (b) selon une quelconque revendication précédente pour ainsi obtenir la composition pharmaceutique, éventuellement l'agent antibactérien, l'agent anti-inflammatoire, l'excipient et le support étant combinés dans un procédé de formulation en un lot.

10. Composition selon l'une quelconque des revendications 1 à 7 pour une utilisation dans un procédé pour le traitement d'une maladie, d'une affection ou d'une pathologie ophtalmologique chez un sujet mammifère qui a besoin d'un tel traitement, comprenant l'administration de la composition selon la revendication 1 au sujet, le procédé d'administration étant choisi dans le groupe constitué par une injection intravitréenne, une injection intraoculaire intracamérale, une injection intra-lésionnelle, une injection intra-articulaire, une injection sous-conjonctive, une injection sous-ténonienne, une administration via des gouttes oculaires, une administration via un spray et une administration intra-canaliculaire, pour traiter ainsi la maladie, l'affection ou la pathologie ophtalmologique.

11. Composition pour une utilisation selon la revendication 10, l'injection intravitréenne étant choisie dans le groupe constitué par une injection transzonulaire et une injection non transzonulaire.

12. Composition pour une utilisation selon la revendication 10, la composition étant injectée de manière transzonulaire intravitréenne.

13. Composition pour une utilisation selon la revendication 12, (i) l'agent antibactérien étant la moxifloxacine ; et/ou le corticostéroïde étant la triamcinolone ou un sel, un hydrate, un solvate, un éther, un ester, un acétal ou un cétal pharmaceutiquement acceptable correspondant ou (ii) la moxifloxacine à une concentration d'environ 1,0 mg/mL ; l'acétonide de triamcinolone à une concentration d'environ 15,0 mg/mL ; et le Poloxamer 407® à une concentration d'environ 1,0 % en masse.

14. Composition selon la revendication 2 pour une utilisation dans un procédé pour le traitement d'une maladie, d'une affection ou d'une pathologie ophtalmologique chez un sujet mammifère qui a besoin d'un tel traitement comprenant une injection de manière intraoculaire du sujet, la composition comprenant
(a) une quantité thérapeutiquement efficace de moxifloxacine ;
(b) une quantité thérapeutiquement efficace acétonide de triamcinolone ; et
(c) une quantité de Poloxamer 407®
pour traiter ainsi la maladie, l'affection ou la pathologie ophtalmologique.

15. Composition pour une utilisation selon la revendication 14, l'injection étant intravitréenne et transzonulaire, ou l'injection étant intraopératoire.

16. Kit pharmaceutique, comprenant un récipient scellé contenant la composition pharmaceutique selon l'une quelconque des revendications 1 à 7 et une instruction pour l'utilisation de la composition enfermée dans le récipient.
